# FASCICULE DE BREVET EUROPEEN

(11) **EP 2 720 252 B1**
(45) Date de publication et mention de la délivrance du brevet: **18.04.2018**
(21) Numéro de dépôt: 13188550.1
(22) Date de dépôt: 14.10.2013
(51) Int. Cl.: H01J 47/02, G01T 1/185

(54) **Detecteur courbe de particules gazeux**
Gekrümmter gasgefüllter Teilchendetektor
Curved gaseous particle detector

(30) Priorité: 15.10.2012 FR 1259799
(43) Date de publication de la demande: 16.04.2014
(73) Titulaire: COMMISSARIAT À L'ÉNERGIE ATOMIQUE ET AUX ÉNERGIES ALTERNATIVES, 75015 Paris (FR)
(72) Inventeur: Cazaux, Sandrine, 91310 Monlhery (FR); Lerch, Thierry, 91140 Alfortville (FR); Aune, Stephan, 91120 Palaiseau (FR)
(74) Mandataire: Lebkiri, Alexandre

(56) Documents cités:
- US-A- 3 703 638
- US-A- 4 583 331
- US-A- 6 011 265
- US-A1- 2012 049 054
- FENKER H ET AL: "BoNus: Development and use of a radial TPC using cylindrical GEMs", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NETHERLANDS, vol. 592, no. 3, 21 juillet 2008 (2008-07-21), pages 273-286, XP022831720, ISSN: 0168-9002, DOI: 10.1016/J.NIMA.2008.04.047 [extrait le 2008-05-10]
- BALLA A ET AL: "Status of the cylindrical-GEM project for the KLOE-2 inner tracker", NUCLEAR INSTRUMENTS & METHODS IN PHYSICS RESEARCH. SECTION A: ACCELERATORS, SPECTROMETERS, DETECTORS, AND ASSOCIATED EQUIPMENT, vol. 628, no. 1, 8 juillet 2010 (2010-07-08), pages 194-198, XP028128048, ISSN: 0168-9002, DOI: 10.1016/J.NIMA.2010.06.315 [extrait le 2010-07-08]
- BENCIVENNI G ET AL: "The full scale prototype of the cylindrical-GEM detector as inner tracker in KLOE2", NUCLEAR SCIENCE SYMPOSIUM CONFERENCE RECORD, 2007. NSS '07. IEEE, IEEE, PI, 1 octobre 2007 (2007-10-01), pages 4666-4670, XP031206623, ISBN: 978-1-4244-0922-8

## Description

### DOMAINE TECHNIQUE

La présente invention se rapporte à un détecteur de particules gazeux, notamment les détecteurs connus sous l'appellation « détecteur micro-mégas » (pour « MICRO MEsh GAseous Structure ») ou les détecteurs connus sous l'appelation GEM (pour « Gaz Electron Multiplier »).

### ETAT DE LA TECHNIQUE

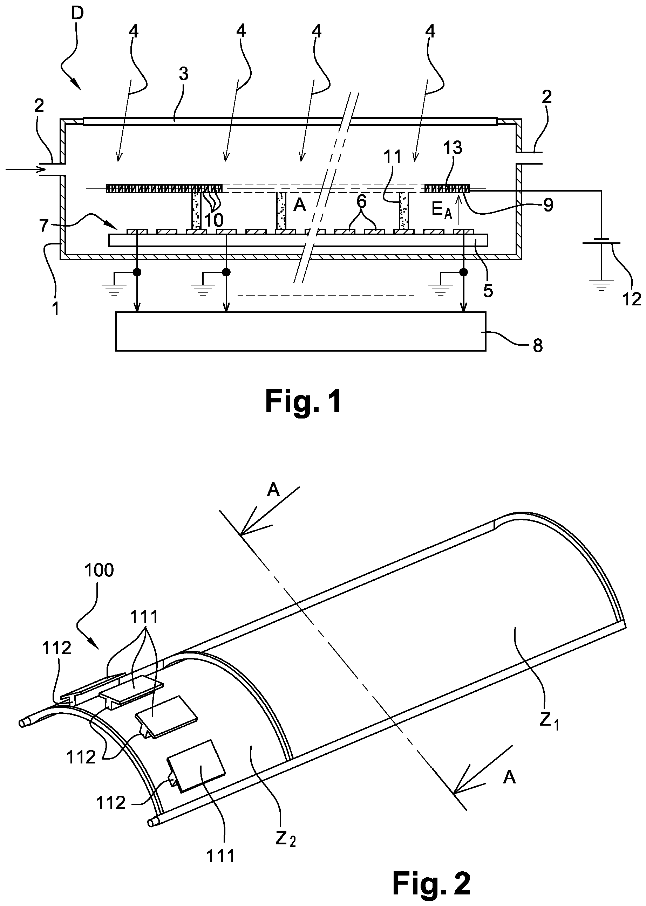

On connaît des détecteurs D de localisation de particules, tel que celui illustré sur la figure 1 comportant une enceinte à gaz 1 qui est remplie d'un mélange gazeux approprié, un tel détecteur D permettant l'amplification d'électrons par un processus d'avalanche. Un tel détecteur est notamment divulgué par le document FR2786024. Dans ce cas particulier, les particules sont des photons.

Le mélange gazeux contenu dans l'enceinte 1 peut rester confiné dans l'enceinte 1 ou circuler au travers d'un purificateur (non illustré) par l'intermédiaire de canalisations 2.

Cette enceinte 1 est fermée de façon étanche par une fenêtre 3 transparente aux photons 4 que l'on veut détecter. Le détecteur D comprend aussi une plaque électriquement isolante 5 de très bonne planéité, sur laquelle est formée la partie active du détecteur D, en particulier des anodes élémentaires 6 qui peuvent être des pistes métalliques parallèles ou des éléments métalliques que l'on peut appeler des « pixels » et qui forment un réseau bidimensionnel sur la plaque électriquement isolante 5. L'ensemble des pistes constitue l'anode 7 du détecteur D. Les pistes sont mises à la masse et sont reliées à des moyens électroniques appropriés 8 prévus pour amplifier puis traiter les signaux électriques provenant de ces pistes.

En outre, cette partie active comprend, en regard de la fenêtre 3, une cathode 9 constituée par une feuille métallique percée de trous 10, cette feuille formant ainsi une grille. L'anode 7 et la grille 9 sont maintenues parallèles l'une à l'autre grâce à des espaceurs 11 électriquement isolants qui reposent sur l'anode 7.

Des moyens de polarisation 12 sont en outre prévus pour porter la grille 9 (c'est-à-dire la cathode) à une tension fortement négative par rapport à l'anode 7 (cette tension dépendant du mélange gazeux utilisé). L'anode 7, qui est ainsi portée à un potentiel élevé par rapport à la cathode 9 constitue avec cette dernière un détecteur à électrodes parallèles capable d'amplifier des électrons par un processus d'avalanche qui se développe entre ces électrodes. Dans l'exemple représenté, la haute tension est choisie pour créer dans l'espace A compris entre l'anode 7 et la cathode 9, ou espace d'amplification, un champ électrique EA dont l'intensité est supérieure ou égale à 50 kV/cm.

Dans le cas de la détection de particules non ionisantes comme les photons, il est nécessaire d'ajouter une couche 13 de conversion pour convertir les particules non ionisantes en particules ionisantes.

Ce type de détecteur de particules présente néanmoins des inconvénients. En effet, lorsque l'on souhaite obtenir une structure courbe, il est nécessaire d'abouter une pluralité de structures planes de façon à obtenir une surface courbée. Cette particularité ne permet en aucun cas d'obtenir un détecteur compact ni même léger. L'article "BoNus: Development and use of a radial TPC using cylindrical GEMs" de Fenker et al, XP022831720, DOI:10.1016/ j.nima.2008.04.047 décrit un détecteur courbe de particules gazeux comportant un empilement de deux couches courbes.

Dans ce contexte, la présente invention a pour but de fournir un détecteur de particules gazeux à la fois compact et léger.

A cette fin, l'invention porte sur un détecteur courbe de particules gazeux comportant un empilement de deux couches. Ces couches sont courbes et maintenues ensemble par un cadre formé de deux longerons définissant un plan, lesdits deux longerons étant reliés entre eux par au moins deux barres courbes hors de ce plan, le cadre étant placé entre les deux couches de l'empilement.

Un tel détecteur courbe présente une structure autoporteuse qui fait partie intégrante du détecteur, les éléments formant le cadre permettent à la fois de réaliser un espace de détection, de maintenir les deux couches solidaires entre elles et de rigidifier le détecteur. Ces particularités avantageuses permettent non seulement de diminuer le poids du détecteur mais également d'augmenter sa compacité.

Le détecteur selon l'invention peut également présenter une ou plusieurs des caractéristiques ci-après, considérées individuellement ou selon toutes les combinaisons techniquement réalisables.

Dans une réalisation non limitative du détecteur selon l'invention, les deux couches sont transparentes à plus de 99% aux particules à détecter.

Dans une réalisation non limitative du détecteur selon l'invention, les deux couches sont formées par :
- une première couche de support, et
- une deuxième couche, dite de dérive,
ladite première couche comportant une face extérieure en regard de ladite deuxième couche, ladite face extérieure supportant une partie active de détection.

Dans une réalisation non limitative du détecteur selon l'invention, la partie active de détection comprend des moyens d'amplification.

Dans une réalisation non limitative du détecteur selon l'invention, le cadre est en un matériau dont le module d'Young est supérieur à 30 GPa.

Dans une réalisation non limitative du détecteur selon l'invention, le cadre est en carbone.

Dans une réalisation non limitative du détecteur selon l'invention, au moins un des longerons est creux de façon à ce que du gaz puisse le traverser, ledit au moins un longeron creux comportant en outre au moins un orifice débouchant dans la zone de détection du détecteur comprise entre les deux couches. Dans cette réalisation, le au moins un longeron creux peut comporter à chacune de ses extrémités un raccord de gaz.

Dans une réalisation non limitative du détecteur selon l'invention, les deux barres courbes sont parallèles entre elles et présentent une courbure continue.

Dans une réalisation non limitative du détecteur selon l'invention, un joint est disposé sur le pourtour dudit détecteur de sorte à assurer l'étanchéité dudit détecteur. Le joint peut par exemple présenter une épaisseur d'au moins 1 mm.

Dans une réalisation non limitative du détecteur selon l'invention, chaque longeron et chaque barre courbe présentent une même épaisseur constante de sorte que les deux couches soient équidistantes.

Dans une réalisation non limitative du détecteur selon l'invention, la face extérieure de la deuxième couche forme une face extérieure de détection d'un autre détecteur disposé au dessus de ladite deuxième couche.

Dans une réalisation non limitative du détecteur selon l'invention, la première couche et la deuxième couche sont positionnées l'une par rapport à l'autre au moyen d'au moins deux pions, chacun desdits deux pions traversant lesdites première et deuxième couches et les barres courbes.

Dans une réalisation non limitative du détecteur selon l'invention, des fiches de connexion électronique sont fixées sur la première couche, chacune des fiches comportant des moyens élastiques de sorte à améliorer le contact électrique entre les fiches et le détecteur.

L'invention a également pour objet une structure de maintien d'une pluralité de détecteurs. Ladite structure comporte notamment :
- une première plaque,
- une deuxième plaque transparente aux particules à détecter, ladite première plaque et ladite deuxième plaque étant reliées entre elles par un système de glissière transparent aux particules à détecter que l'on veut détecter, ledit système de glissière étant adapté pour recevoir une pluralité de détecteurs selon l'invention.

D'autres caractéristiques et avantages de l'invention ressortiront clairement de la description qui en est donnée ci-dessous, à titre indicatif et nullement limitatif, en référence aux figures annexées, parmi lesquelles :
- la figure 1 illustre schématiquement un détecteur de localisation de particules à remplissage gazeux selon l'état de la technique ;
- la figure 2 illustre un mode de réalisation non limitatif d'un détecteur courbe de particules gazeux conforme à l'invention ;
- la figure 3 illustre une vue en coupe d'un détecteur courbe de particules gazeux conforme à celui qui est représenté sur la figure 2 ;
- la figure 4 illustre un mode de réalisation non limitatif d'un cadre d'un détecteur courbe de particules gazeux conforme à celui illustré sur la figure 2 ;
- la figure 5 illustre un mode de réalisation d'une fiche de connexion que comporte un détecteur courbe de particules gazeux conforme à celui illustré sur la figure 2 ;
- la figure 6 illustre un mode de réalisation de deux détecteurs courbes de particules gazeux disposés l'un au dessus de l'autre, chaque détecteur courbe de particules étant conforme à celui illustré sur la figure 2 ;
- la figure 7 illustre un mode de réalisation non limitatif d'une structure de maintien d'une pluralité de détecteurs courbes conformes à celui illustré sur la figure 2.

Dans toutes les figures, les éléments communs portent les mêmes numéros de référence.

La figure 1 a déjà été détaillée pour illustrer un détecteur de localisation de particules à remplissage gazeux selon l'état de la technique.

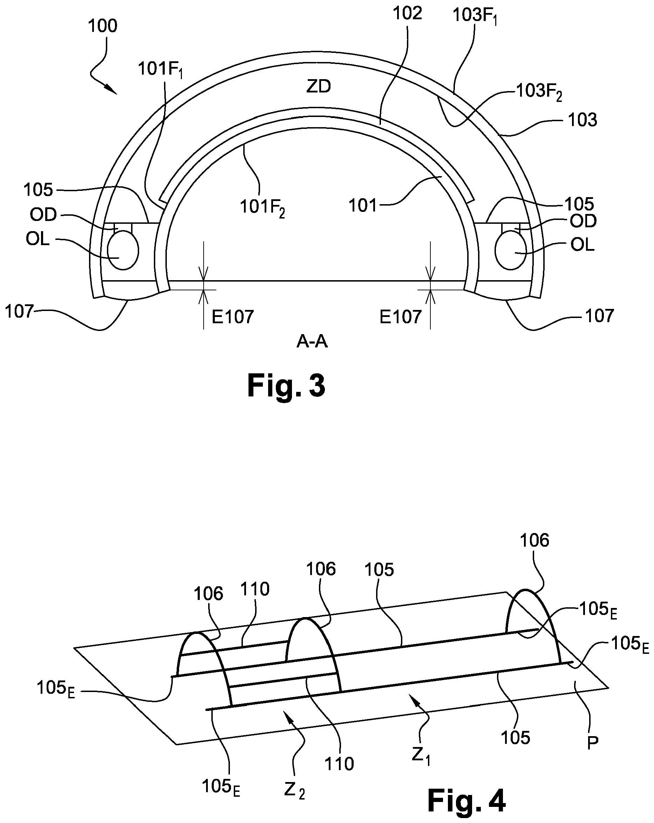

La figure 2 illustre un premier mode de réalisation non limitatif d'un détecteur courbe de particules gazeux 100 également dénommé détecteur micro-mégas « pour MICRO MEsh GAseous Structure en anglais » conforme à celui de l'invention. La figure 3 est une vue en coupe selon A-A du détecteur courbe de particules gazeux 100 illustré sur la figure 2.

Le détecteur de particules gazeux 100 comporte un empilement de deux couches 101 et 103. Ces couches 101 et 103 sont courbes et sont maintenues ensemble par un cadre 104 conforme à celui illustré sur la figure 4. On notera que la cadre 104 est positionné entre les deux couches 101 et 103 de l'empilement.

Dans l'exemple de réalisation du cadre 104 illustré sur la figure 4, le cadre 104 comporte deux longerons 105 (ou barres droites) et trois barres courbes 106 (ou arceaux). Les deux longerons 105 définissent un plan P, et sont reliés entre eux au moyen des trois barres courbes 106. On notera que les trois barres courbes 106 sont hors de ce plan P. Le rayon de courbure des barres courbes 106 dépend de la courbure souhaitée.

Le matériau du cadre 104 peut être de façon non limitative du carbone. D'autres matériaux sont envisageables, il est préférable que ces matériaux présentent un module d'Young supérieur 30 GPa afin de présenter une rigidité suffisante pour être autoportant. Le kevlar, l'aluminium ou encore l'inox sont des matériaux présentant de telles caractéristiques.

Le carbone est avantageux pour ses qualités tant en termes de performances mécaniques que physiques. En effet, ce matériau a une densité faible (environ 1900 kg/m3), un module d'Young élevé (environ 110 GPa), une contrainte à la rupture élevée (environ 1000MPa) et une longueur de radiation élevée (environ 302 mm). Le carbone permet donc de réaliser un cadre 104 à la fois rigide, léger et transparent aux particules.

Dans cette réalisation, les longerons 105 sont creux de façon à ce que du gaz puisse les traverser. Comme illustré sur la vue en coupe A-A, les longerons 105 sont traversés dans leur longueur par un orifice longitudinal OL. Chaque longeron 105 présente également des orifices débouchant OD (seulement deux sont représentés) à la fois dans l'orifice longitudinal OL et dans la zone de détection ZD du détecteur 100. Cette zone de détection ZD est comprise entre les deux couches 101 et 103. Les orifices longitudinal OL et débouchant OD permettent d'acheminer le gaz, via les longerons 105, dans le détecteur 100. Chaque longeron 105 creux comporte à chacune de ses extrémités 105^{E} un raccord de gaz (non illustré). Ces raccords de gaz peuvent par exemple être insérés et collés dans les longerons 105, plus particulièrement insérés et collés dans les orifices longitudinaux OL.

Dans un exemple non limitatif, les deux longerons 105 présentent une section carrée de dimension 3 x 3 mm et une longueur L de 658 mm.

On notera que les trois barres courbes 106 sont parallèles entre elles et présentent une courbure continue. Ces trois barres courbes 106 présentent par exemple une épaisseur de 3 mm, soit une épaisseur identique à celle des longerons 105. En d'autres termes, les deux couches 101 et 103 sont équidistantes.

Ces trois barres courbes 106 présentent en outre un rayon de courbure déterminé.

Les deux couches 101 et 103 que comporte le détecteur 100 de particules gazeux sont formées par :
- une première couche 101 de support, et
- une deuxième couche 103, dite de dérive.

La première couche 101 de support est formée par exemple, par une feuille de type Kapton™, PCB, PCB multicouches ou autre présentant une épaisseur, par exemple de 200 µm. La première couche 101 comporte une face extérieure 101F1 en regard de la deuxième couche 103. Cette face extérieure 101F1 supporte une partie active de détection 102. Cette partie active de détection 102 peut comporter des moyens d'amplification. Ces moyens d'amplification sont par exemple :
- une grille pour les détecteurs Micromegas,
- un empilement de feuillets d'amplification pour les détecteurs GEM.

La face intérieure 101F2 de la première couche 101 peut supporter des pistes de connexion ou être métallisée pour servir de blindage électromagnétique.

La deuxième couche 103 est, par exemple, formée par une feuille de type Kapton™, PCB, Mylar™. Cette deuxième couche 103 présente par exemple une épaisseur de l'ordre de 200µm. Cette deuxième couche 103 doit présenter des propriétés de conduction pour établir le potentiel de dérive. Elle peut être en un matériau quelconque dont :
- la face extérieure 103F1 peut être recouverte d'un matériau conducteur servant de blindage électromagnétique, et
- la face intérieure 103F2 est recouverte d'un matériau conducteur pour constituer la couche de dérive.

De façon générale, l'épaisseur des couches sera déterminée en fonction du module d'Young du matériau utilisé. Il faudra qu'elle soit assez fine pour que le matériau puisse être courbé et assez élevée pour que la structure ne s'effondre pas sur elle-même. Par exemple, pour une structure de dimensions de l'ordre de 50 cm (longueur des longerons) des couches en PCB seront comprises entre 60 et 100 µm et des couches en Kapton seront comprises entre 100 et 150 µm.

On pourra noter que le cadre 104, lequel est disposé entre les deux couches 101 et 103, forme une entretoise de dérive. Le cadre 104 en carbone est positionné et collé sur la première couche 101, et la deuxième couche 103 est positionnée et collée sur le cadre 104 en carbone.

Dans l'exemple décrit, chaque barre courbe 106 et chaque longeron 105 présente une épaisseur de l'ordre 3 mm de façon à espacer la première couche 101 et la deuxième couche 103 d'une distance de 3 mm, cette distance étant nécessaire pour réaliser une détection de particules.

Il est entendu qu'une telle distance n'est pas limitative et que le cadre 104 peut présenter une épaisseur supérieure ou inférieure à 3 mm.

Dans une réalisation non limitative, un joint 107 est disposé sur le pourtour du détecteur 100 de sorte à assurer l'étanchéité du détecteur 100. Le joint 107 peut par exemple être formé par une bourde de colle disposée sur le pourtour du détecteur 100 entre la première couche 101 et la deuxième couche 103. Cette bourde de colle formant le joint 107 peut présenter une épaisseur E107 d'au moins 1 mm. Cette colle est avantageusement isolante, résistante et transparente aux particules à détecter.

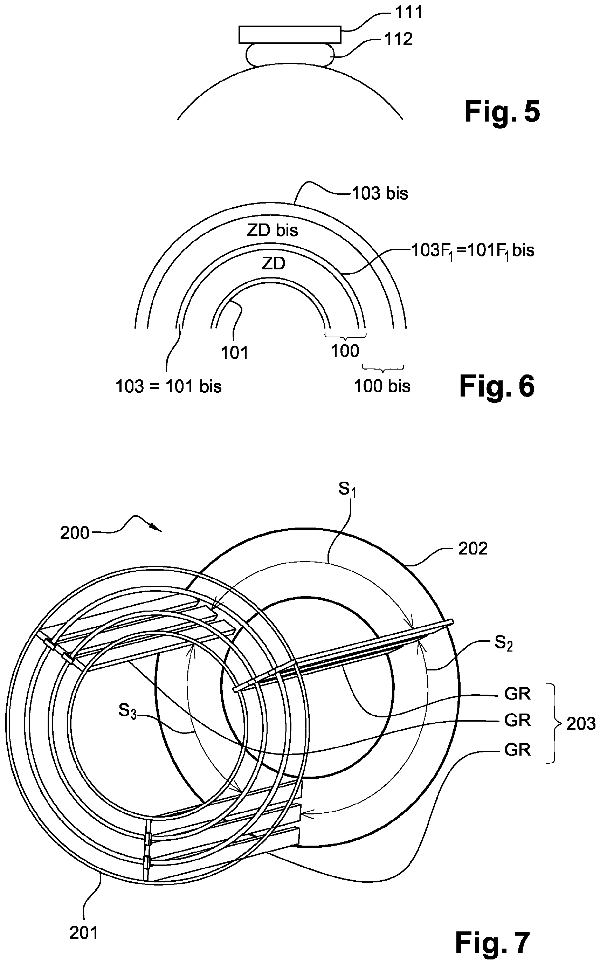

En outre, le détecteur 100 comporte une zone active Z1 et une zone de connexion Z2. La zone de connexion Z2 est rigidifiée par exemple par d'autres longerons 110 collés sur la première couche 101 et reliant les barres courbes 106 (voir figure 4) de façon à pouvoir monter et démonter des connecteurs sans pour autant fragiliser le détecteur 100. Cette zone de connexion Z2 comporte des fiches 111 de connexion électronique, chacune des fiches 111 comporte des moyens élastiques 112 de sorte à améliorer les contacts entre le détecteur 100 et la fiche 111 sans contraindre la couche 101 pour compenser le rayon de courbure (voir figure 5).

Dans une réalisation de l'invention illustrée sur la figure 6, la face extérieure 103F1 de la deuxième couche 103 forme une face 101F1 bis de détection d'un autre détecteur 100 bis disposé au dessus de la deuxième couche 103 du détecteur 100. En d'autres termes, selon cette réalisation la deuxième couche 103 d'un détecteur 100 fait également office de première couche 101 bis d'un autre détecteur 100bis.

L'invention a également pour objet une structure de maintien 200 d'une pluralité de détecteurs 100 selon l'invention, une telle structure de maintien 200 étant représentée de façon schématique sur la figure 7.

En effet, si le détecteur présente un rayon de courbure très élevé et/ou une surface très grande, il ne sera probablement pas possible de réaliser un détecteur cylindrique : il sera alors constitué d'un assemblage de plusieurs secteurs de détecteurs, par exemple ici 3 (chaque secteur de détecteurs étant formé par un détecteur 100 conforme à l'invention).

La structure de maintien 200 comporte une première plaque 201 et une deuxième plaque 202, cette deuxième plaque 202 étant transparente aux particules que l'on veut détecter.

La première plaque 201 et la deuxième plaque 202 sont reliées entre elles par un système de glissière 203 également transparent aux particules que l'on veut détecter, le système de glissière 203 étant adapté pour recevoir une pluralité de détecteurs 100 conforme à l'invention. Le matériau du système de glissière 203 est avantageusement du carbone. Comme le système de glissière 203 est situé dans la zone de détection, ce système 203 est transparent aux particules à détecter.

Dans cet exemple non limitatif, le système de glissière 203 est formé par trois groupes de rails GR. Ces groupes de rails GR divisent le cylindre de la structure de maintien 200 en trois secteurs S1, S2, S3, chacun des secteurs S1, S2 et S3 étant apte à recevoir six détecteurs 100 selon l'invention. Pour ce faire, les six détecteurs 100 sont coulissés entre deux groupes de rails. Ces six détecteurs 100 ainsi positionnés sont superposés les uns aux autres dans un même secteur. En d'autres termes, une telle structure de maintien 200 est adaptée pour maintenir dix-huit détecteurs 100 conformes à l'invention.

La première plaque 201 située à une extrémité de la structure 200 est par exemple en aluminium et est ajourée de façon à autoriser le montage et le démontage des détecteurs 100. En outre, cette première plaque 201 est ajourée de façon à diminuer le poids de la structure 200.

A l'opposé, la deuxième plaque 202 est, de façon non limitative, pleine et est par exemple en carbone transparent aux particules que l'on veut détecter (cette transparence est nécessaire puisqu'elle est située au niveau de la zone de détection de la structure 200). Cette deuxième plaque 202 obstrue une des extrémités de la structure 200 en assurant l'étanchéité de celle-ci.

En outre, la première plaque 201 et la deuxième plaque 202 rigidifient la structure de maintien 200.

On notera que la structure de maintien 200 permet d'insérer, de positionner et de supporter par exemple six couches de détecteurs par secteur S1, S2, S3.

En outre, la structure de maintien 200 peut également supporter une pluralité de détecteurs de particules gazeux plans. Dans ce cas, les détecteurs plans sont positionnés dans la zone de détection et au niveau de la deuxième plaque 202.

En d'autres termes, cette structure de maintien 200 selon l'invention permet le positionnement précis de tous les types de détecteur (détecteurs courbes et plans) pour constituer des cylindres constituant la structure de maintien 200 et de rigidifier l'ensemble pour obtenir peu de déformation de l'ensemble et des détecteurs de façon à pouvoir positionner précisément et toujours à la même position les détecteurs (dans le cas d'un changement de détecteurs 100 défectueux par exemple).

En outre, du fait de l'utilisation du carbone, un minimum de matière, de zones mortes sont présentes. Le carbone allie légèreté, transparence, bonne propriété mécanique et isolation thermique.

On notera par ailleurs que l'utilisation de détecteurs courbes permet d'augmenter la zone de détection.

On notera en outre que chaque détecteur courbe 100 présente une structure autoporteuse. En effet, les éléments le composant sont collés entre eux (gain de place), le gaz passe dans la mécanique (gain de poids, de volume de matière). Le détecteur 100 est compact et les zones mortes sont limitées.

## Revendications

1. Détecteur courbe de particules gazeux (100) comportant un empilement de deux couches courbes (101, 103), **caractérisé en ce que** lesdites deux couches (101, 103) sont maintenues ensemble par un cadre (104) formé de deux longerons (105) définissant un plan (P), lesdits deux longerons (105) étant reliés entre eux par au moins deux barres courbes (106) hors de ce plan, le cadre (104) étant placé entre lesdites deux couches (101, 103) de l'empilement.

2. Détecteur courbe de particules gazeux (100) selon la revendication précédente, **caractérisé en ce que** les deux couches (101, 103) sont transparentes à plus de 99% aux particules à détecter.

3. Détecteur courbe de particules gazeux (100) selon l'une quelconque des revendications précédentes, **caractérisé en ce que** les deux couches (101, 103) sont formées par :
- une première couche (101) de support, et
- une deuxième couche (103), dite de dérive,
ladite première couche (101) comportant une face extérieure (101F1) en regard de ladite deuxième couche (103), ladite face extérieure (101F1) supportant une partie active de détection (102).

4. Détecteur courbe de particules gazeux (100) selon la revendication 3 précédente, **caractérisé en ce que** la partie active de détection (102) comprend des moyens d'amplification.

5. Détecteur courbe de particules gazeux (100) selon l'une quelconque des revendications précédentes, **caractérisé en ce que** le cadre (104) est en un matériau dont le module d'Young est supérieur à 30 GPa.

6. Détecteur courbe de particules gazeux (100) selon l'une quelconque des revendications précédentes, **caractérisé en ce que** le cadre (104) est en carbone.

7. Détecteur courbe de particules gazeux (100) selon l'une quelconque des revendications précédentes, **caractérisé en ce qu'**au moins un des longerons (105) est creux de façon à ce que du gaz puisse le traverser, ledit au moins un longeron (105) creux comportant en outre au moins un orifice débouchant (OD) dans la zone de détection (ZD) du détecteur (100) comprise entre les deux couches (101, 103).

8. Détecteur courbe de particules gazeux (100) selon l'une quelconque des revendications précédentes, **caractérisé en ce que** les deux barres courbes (106) sont parallèles entre elles et présentent une courbure continue.

9. Détecteur courbe de particules gazeux (100) selon l'une quelconque des revendications précédentes, **caractérisé en ce qu'**un joint (107) est disposé sur le pourtour dudit détecteur (100) de sorte à assurer l'étanchéité dudit détecteur (100).

10. Détecteur courbe de particules gazeux (100) selon l'une quelconque des revendications précédentes, **caractérisé en ce que** chaque longeron (105) et chaque barre courbe (106) présentent une même épaisseur constante de sorte que les deux couches (101, 103) soient équidistantes.

11. Détecteur courbe de particules gazeux (100) selon l'une quelconque des revendications précédentes, **caractérisé en ce que** la face extérieure (103F1) de la deuxième couche (103) forme une face extérieure (101F1bis) de détection d'un autre détecteur (100bis) disposé au dessus de ladite deuxième couche (103).

12. Détecteur courbe de particules gazeux (100) selon l'une quelconque des revendications précédentes, **caractérisé en ce que** la première couche (101) et la deuxième couche (103) sont positionnées l'une par rapport à l'autre au moyen d'au moins deux pions, chacun desdits deux pions traversant lesdites première et deuxième couches (101, 103) et les barres courbes (106).

13. Détecteur courbe de particules gazeux (100) selon l'une quelconque des revendications précédentes, **caractérisé en ce que** des fiches (111) de connexion électronique sont fixées sur la première couche (101), chacune des fiches (111) comportant des moyens élastiques (112) de sorte à améliorer le contact électrique entre les fiches (111) et le détecteur (100).

14. Structure de maintien (200) d'une pluralité de détecteurs, ladite structure (200) étant **caractérisée en ce qu'**elle comporte :
- une première plaque (201),
- une deuxième plaque (202) transparente aux particules à détecter,
ladite première plaque (201) et ladite deuxième plaque (202) étant reliées entre elles par un système de glissière (203) transparent aux particules à détecter que l'on veut détecter, ledit système de glissière (203) étant adapté pour recevoir une pluralité de détecteurs courbe (100) selon l'une quelconque des revendications précédentes.

## Patentansprüche

1. Gekrümmter Detektor gasförmiger Partikel (100), umfassend eine Stapelung von zwei Schichten Krümmungen (101, 103), **dadurch gekennzeichnet, dass** die genannten zwei Schichten (101, 103) durch einen Rahmen (104) zusammengehalten sind, der aus zwei Längsträgern (105) gebildet ist, der eine Ebene (P) definiert, wobei die genannten zwei Längsträger (105) miteinander durch wenigstens zwei gekrümmte Stange (106) außerhalb dieser Ebene verbunden sind, wobei der Rahmen (104) zwischen den genannten zwei Schichten (101, 103) der Stapelung platziert ist.

2. Gekrümmter Detektor gasförmiger Partikel (100) gemäß dem voranstehenden Anspruch, **dadurch gekennzeichnet, dass** die zwei Schichten (101, 103) zu mehr als 99 % für die zu detektierenden Partikel transparent sind.

3. Gekrümmter Detektor gasförmiger Partikel (100) gemäß irgendeinem der voranstehenden Ansprüche, **dadurch gekennzeichnet, dass** die zwei Schichten (101, 103) geformt sind durch:
- eine erste Trägerschicht (101) und
- eine zweite Schicht (103), bezeichnet als Ableitschicht,
wobei die genannte erste Schicht (101) eine äußere Seite (101F1) gegenüber der genannten zweiten Schicht (103) umfasst, wobei die genannte äußere Seite (101F1) einen aktiven Detektionsteil (102) trägt.

4. Gekrümmter Detektor gasförmiger Partikel (100) gemäß dem voranstehenden Anspruch 3, **dadurch gekennzeichnet, dass** der aktive Detektionsteil (102) Verstärkungsmittel umfasst.

5. Gekrümmter Detektor gasförmiger Partikel (100) gemäß irgendeinem der voranstehenden Ansprüche, **dadurch gekennzeichnet, dass** der Rahmen (104) aus einem Material ist, dessen Young-Modell größer ist als 30 GPa.

6. Gekrümmter Detektor gasförmiger Partikel (100) gemäß irgendeinem der voranstehenden Ansprüche, **dadurch gekennzeichnet, dass** der Rahmen (104) aus Kohlenstoff ist.

7. Gekrümmter Detektor gasförmiger Partikel (100) gemäß irgendeinem der voranstehenden Ansprüche, **dadurch gekennzeichnet, dass** wenigstens einer der Längsträger (105) derart hohl ist, dass Gas ihn durchqueren kann, wobei der wenigstens eine hohle Längsträger (105) darüber hinaus wenigstens eine Öffnung (OD) umfasst, die in den Detektionsbereich (ZD) des Detektors (100) mündet, die zwischen den zwei Schichten (101, 103) inbegriffen ist.

8. Gekrümmter Detektor gasförmiger Partikel (100) gemäß irgendeinem der voranstehenden Ansprüche, **dadurch gekennzeichnet, dass** die zwei gekrümmten Stangen (106) zueinander parallel sind und eine kontinuierliche Krümmung aufweisen.

9. Gekrümmter Detektor gasförmiger Partikel (100) gemäß irgendeinem der voranstehenden Ansprüche, **dadurch gekennzeichnet, dass** eine Dichtung (107) auf dem Umfang des genannten Detektors (100) derart angeordnet ist, dass die Abdichtung des genannten Detektors (100) gewährleistet ist.

10. Gekrümmter Detektor gasförmiger Partikel (100) gemäß irgendeinem der voranstehenden Ansprüche, **dadurch gekennzeichnet, dass** jeder Längsträger (105) und jede gekrümmte Stange (106) eine und dieselbe konstante Dicke derart aufweist, dass die zwei Schichten (101, 103) in gleicher Entfernung beabstandet sind.

11. Gekrümmter Detektor gasförmiger Partikel (100) gemäß irgendeinem der voranstehenden Ansprüche, **dadurch gekennzeichnet, dass** die äußere Seite (103F1) der zweiten Schicht (103) eine äußere Detektionsseite (101Fbis) eines anderen Detektors (100bis) bildet, der oberhalb der genannten zweiten Schicht (103) angeordnet ist.

12. Gekrümmter Detektor gasförmiger Partikel (100) gemäß irgendeinem der voranstehenden Ansprüche, **dadurch gekennzeichnet, dass** die erste Schicht (101) und die zweite Schicht (103) im Verhältnis zueinander mittels wenigstens zweier Stifte positioniert sind, wobei jeder der genannten zwei Stifte die genannte erste und zweite Schicht (101, 103) und die gekrümmten Stangen (106) durchquert.

13. Gekrümmter Detektor gasförmiger Partikel (100) gemäß irgendeinem der voranstehenden Ansprüche, **dadurch gekennzeichnet, dass** elektronische Anschlussstecker (111) auf der ersten Schicht (101) befestigt sind, wobei jeder der Stecker (111) elastische Mittel (112) derart umfasst, dass der elektrische Kontakt zwischen den Steckern (111) und dem Detektor (100) verbessert wird.

14. Haltestruktur (200) einer Vielzahl von Detektoren, wobei die genannte Struktur (200) **dadurch gekennzeichnet ist, dass** sie umfasst:
- eine erste Platte (201),
- eine zweite Platte (202), die für die zu detektierenden Partikel transparent ist,
wobei die genannte erste Platte (201) und die genannte zweite Platte (202) miteinander durch ein Gleitschienensystem (203) verbunden sind, das für die zu detektierenden Partikel transparent ist, die detektiert werden sollen, wobei das genannte Gleitschienensystem (203) geeignet ist, um eine Vielzahl von gekrümmten Detektoren (100) gemäß irgendeinem der voranstehenden Ansprüche aufzunehmen.

## Claims

1. Curved gaseous particle detector (100) comprising a stack of two curved layers (101, 103), **characterised in that** said two layers (101, 103) are maintained together by a frame (104) formed of two spars (105) defining a plane (P), said two spars (105) being connected together by at least two curved bars (106) outside of said plane, the frame (104) being placed between said two layers (101, 103) of the stack.

2. Curved gaseous particle detector (100) according to the preceding claim, **characterised in that** the two layers (101, 103) are transparent to more than 99% of the particles to be detected.

3. Curved gaseous particle detector (100) according to any of the previous claims, **characterised in that** the two layers (101, 103) are formed of:
- a first support layer (101), and
- a second layer (103), known as drift layer,
said first layer (101) comprising an outer face (101F1) facing said second layer (103), said outer face (101F1) supporting an active detection part (102) .

4. Curved gaseous particle detector (100) according to the preceding claim 3, **characterised in that** the active detection part (102) comprises amplification means.

5. Curved gaseous particle detector (100) according to any of the previous claims, **characterised in that** the frame (104) is made of a material of which the Young's modulus is greater than 30 GPa.

6. Curved gaseous particle detector (100) according to any of the previous claims, **characterised in that** the frame (104) is made of carbon.

7. Curved gaseous particle detector (100) according to any of the previous claims, **characterised in that** at least one of the spars (105) is hollow so that gas can pass through it, said at least one hollow spar (105) further comprising at least one orifice emerging (OD) into the detection zone (ZD) of the detector (100) comprised between the two layers (101, 103) .

8. Curved gaseous particle detector (100) according to any of the previous claims, **characterised in that** the two curved bars (106) are parallel to each other and have a continuous curve.

9. Curved gaseous particle detector (100) according to any of the previous claims, **characterised in that** a joint (107) is arranged on the perimeter of said detector (100) so as to assure the sealing of said detector (100).

10. Curved gaseous particle detector (100) according to any of the preceding claims, **characterised in that** each spar (105) and each curved bar (106) have a same constant thickness such that the two layers (101, 103) are equidistant.

11. Curved gaseous particle detector (100) according to any of the previous claims, **characterised in that** the outer face (103F1) of the second layer (103) forms an outer detection face (101F1bis) of another detector (100bis) arranged above said second layer (103).

12. Curved gaseous particle detector (100) according to any of the previous claims, **characterised in that** the first layer (101) and the second layer (103) are positioned with respect to each other by means of at least two pins, each of said two pins passing through said first and second layers (101, 103) and the curved bars (106).

13. Curved gaseous particle detector (100) according to any of the previous claims, **characterised in that** electronic connection plugs (111) are fixed on the first layer (101), each of the plugs (111) comprising elastic means (112) so as to improve the electrical contact between the plugs (111) and the detector (100) .

14. Structure for maintaining (200) a plurality of detectors, said structure (200) being **characterised in that** it comprises:
- a first plate (201),
- a second plate (202) transparent to the particles to be detected,
said first plate (201) and said second plate (202) being connected together by a slide system (203) transparent to the particles to be detected that it is wished to detect, said slide system (203) being adapted to receive a plurality of curved detectors (100) according to any of the preceding claims.
